# EUROPEAN PATENT APPLICATION

(11) **EP 4 180 817 A1**
(43) Date of publication of application: **17.05.2023**
(21) Application number: 21838558.1
(22) Date of filing: 14.05.2021
(51) Int. Cl.: G01N 33/94, G01N 33/52, G01N 21/78

(54) **REAGENT COMPOSITION FOR DETECTING ILLICIT DRUGS AND SHEET KIT FOR DETECTING ILLICIT DRUGS COMPRISING SAME**

(30) Priority: 10.07.2020 KR 20200085447; 05.02.2021 KR 20210016939
(71) Applicant: Korea Research Institute of Bioscience and Biotechnology, Daejeon 34141 (KR)
(72) Inventor: LIM, Eun Kyung, Yuseong-g Daejeon 34141 (KR); JANG, Soo Jin, Daejeon 34141 (KR); SON, Seong Uk, Daejeon 34141 (KR); KANG, Byung Hoon, Daejeon 34141 (KR); KANG, Tae Joon, Daejeon 34141 (KR); LEE, Kyu Sun, Daejeon 34141 (KR); JUNG, Ju Yeon, Daejeon 34141 (KR)
(74) Representative: Boult Wade Tennant LLP
(86) International application number: PCT/KR2021/006081
(87) International publication number: WO 2022/010089

(57) **Abstract**

The present disclosure provides a reagent composition for detecting illicit drugs, the composition comprising: a diacetylene derivative compound represented by chemical formula 1; and at least one compound selected from among gabazine (SR-95531), an amine group-linked diacetylene derivative represented by chemical formula 2-1, and a gabazine-linked diacetylene derivative represented by chemical formula 2-2.

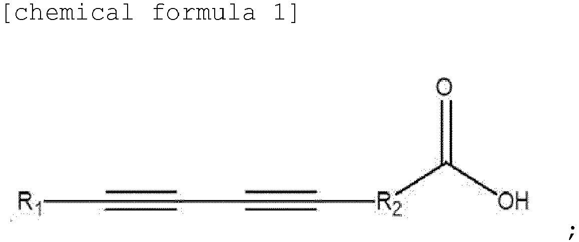

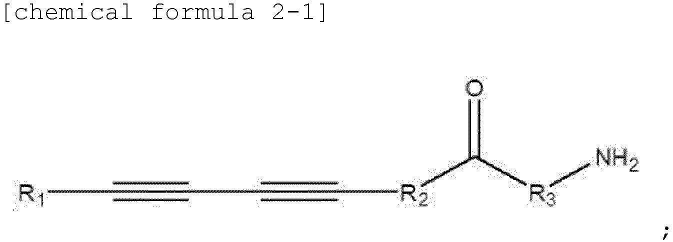

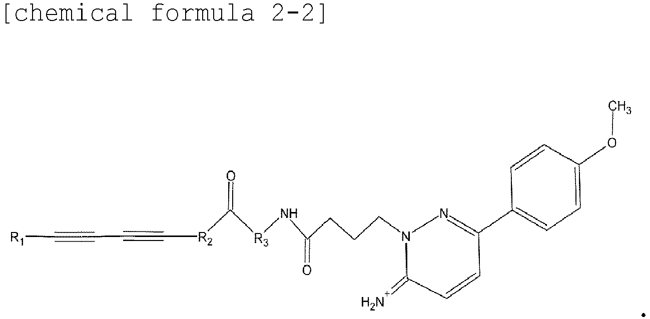

## Description

### [Technical Field]

The present disclosure relates to a reagent composition for detecting illicit drugs abused for sexual crimes and a sheet kit for detecting illicit drugs comprising the same.

### [Background Art]

Illicit drugs refer to narcotics or drugs used for illegal purposes other than medicinal purposes, and generally refer to narcotic drugs. Narcotics act directly on the nerves of the user, resulting in analgesic, anesthetic, excitatory, and hallucinogenic effects. Excessive use of drugs may cause serious harm to the individual's mind and body, and further, may cause serious social controversy.

Under the current law, narcotics are classified into natural drugs, synthetic drugs, psychoactive substances, cannabis, and inhalants. Specifically, the natural drugs include opium, morphine, heroin, codeine, cocaine, and the like, the synthetic drugs include methadone and pethidine hydrochloride; the psychoactive substances include methamphetamine (philopon), barbiturate compounds, and benzodiazepine compounds, LSD, mescaline, and the like; and the inhalants include sniffing glue, butane gas, and the like. Among them, the methamphetamine (philopon) may be produced by a relatively simple method, and cannabis accounts for about 30% of all drugs used in Asia since it is relatively easy to purchase.

In recent years, the number of drug users is increasing day by day in Korea, and accordingly, the need for effective preventive measures and effective treatment methods for habitual drug users is increasing day by day. In particular, the rate of increase in the occurrence of violent crimes such as sexual crimes using illicit drugs such as narcotics is rapidly increasing, emerging as a major social problem. Thus, in order to prevent and reduce these damages, a method of accurately identifying illicit drugs in a simple way is constantly being researched.

In general, it is known that drugs are excreted intact or as partial metabolites in the urine of people who have taken drugs, and there is a correlation between the intake time of the drug and the amount excreted in urine, and thus urine is able to be used to test for drug use. As a recent drug test method, chromatographic immunodetection strips have been used. In the method for detecting a drug using the immunodetection strip, as the sample to be tested moves along the membrane by capillarity, when a drug to be detected is present, the drug reacts with drug antibody-colored microparticles to form a complex and exhibits a competitive reaction with a drug protein conjugate bound to the membrane, and as a result, a color appears in the relevant area, thereby making it possible to determine the presence or absence of the drug. However, there is still a problem in that the detection accuracy is lowered due to problems that respective microparticles do not have uniform sizes, and the movement and reaction of the microparticles are not uniform, and the like.

Therefore, there is a constant need to develop a simple and more accurate method for identifying various types of illicit drugs. In this regard, research on a sensing composition using a conjugated polymer, which has been studied the most in the field of sensing applications, instead of using the colored microparticles, is ongoing.

The present inventors have developed a novel reagent composition capable of quickly and simply detecting illicit drugs, detecting various types of drugs, and having excellent detection accuracy.

### [Related Art Documents]

(Patent Document 1) Korean Patent Laid-Open Publication No. 10-2011-0106513
(Non-Patent Document 1) Materials (Basel). 2016 Mar 16; 9 (3)

### [Disclosure]

### [Technical Problem]

An object of the present disclosure is to provide a reagent composition for detecting illicit drugs.

Another object of the present disclosure is to provide a sheet kit comprising the reagent composition for detecting illicit drugs.

Still another object of the present disclosure is to provide a method for producing a sheet kit for detecting illicit drugs.

### [Technical Solution]

The present disclosure will be described in detail as follows. Meanwhile, each description and embodiment disclosed in the present disclosure may be applied to each of the other descriptions and embodiments. In other words, all combinations of various elements disclosed in the present disclosure fall within the scope of the present disclosure. In addition, it cannot be considered that the scope of the present disclosure is limited by specific descriptions described below.

In one general aspect, the present disclosure provides a reagent composition for detecting illicit drugs, comprising: a diacetylene derivative compound represented by the following Chemical Formula 1; and at least one compound selected from the group consisting of gabazine (SR-95531), an amine group-linked diacetylene derivative represented by the following Chemical Formula 2-1, and a gabazine-linked diacetylene derivative represented by the following Chemical Formula 2-2:

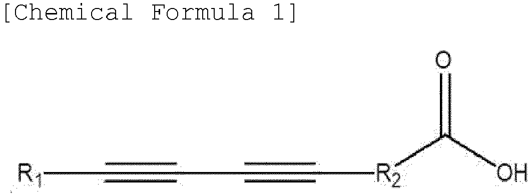

in Chemical Formula above, R₁ is a C₁-C₁₅ alkyl group and R₂ is a C₁-C₁₀ alkylene group, preferably R₁ is a C₆-C₁₃ alkyl group and R₂ is a C₄-C₉ alkylene group, and more preferably R₁ is a C₁₀-C₁₂ alkyl group and R₂ is a C₆-C₈ alkylene group,

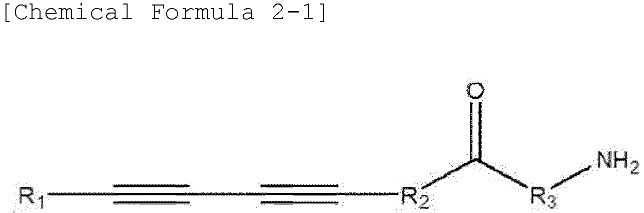

in Chemical Formula above, R₁ is a C₁-C₁₅ alkyl group, R₂ is a C₁-C₁₀ alkylene group, and R₃ is -NH-C₁-C₆ alkylene group or a C₁-C₆ alkylene group, preferably R₁ is a C₆-C₁₃ alkyl group, R₂ is a C₄-C₉ alkylene group, and R₃ is -NH-C₁-C₄ alkylene group, and more preferably R₁ is a C₁₀-C₁₂ alkyl group, R₂ is a C₆-C₈ alkylene group, and R₃ is -NH-C₁-C₃ alkylene group, 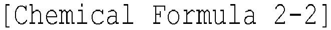 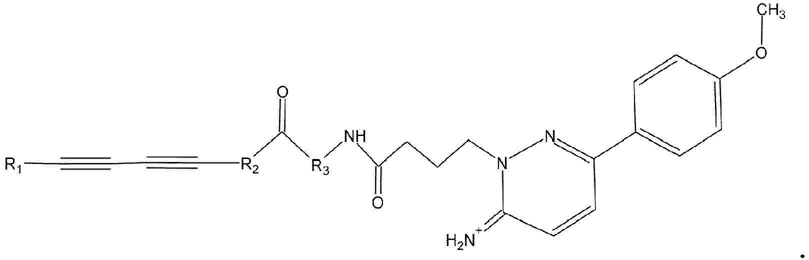 in Chemical Formula above, R₁ is a C₁-C₁₅ alkyl group, R₂ is a C₁-C₁₀ alkylene group, and R₃ is -NH-C₁-C₆ alkylene group or a C₁-C₆ alkylene group, preferably R₁ is a C₆-C₁₃ alkyl group, R₂ is a C₄-C₉ alkylene group, and R₃ is -NH-C₁-C₄ alkylene group, and more preferably R₁ is a C₁₀-C₁₂ alkyl group, R₂ is a C₆-C₈ alkylene group, and R₃ is -NH-C₁-C₃ alkylene group.

In another general aspect, the present disclosure provides a reagent composition for detecting illicit drugs, comprising: a diacetylene derivative compound represented by the following Chemical Formula 1; at least one compound selected from the group consisting of gabazine (SR-95531), an amine group-linked diacetylene derivative represented by the following Chemical Formula 2-1, and a gabazine-linked diacetylene derivative represented by the following Chemical Formula 2-2; a polyethylene glycol-based compound; and a thermoplastic fluorine polymer compound:

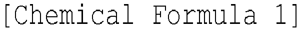

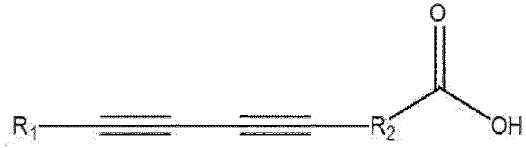

in Chemical Formula above, R₁ is a C₁-C₁₅ alkyl group and R₂ is a C₁-C₁₀ alkylene group, preferably R₁ is a C₆-C₁₃ alkyl group and R₂ is a C₄-C₉ alkylene group, and more preferably R₁ is a C₁₀-C₁₂ alkyl group and R₂ is a C₆-C₈ alkylene group,

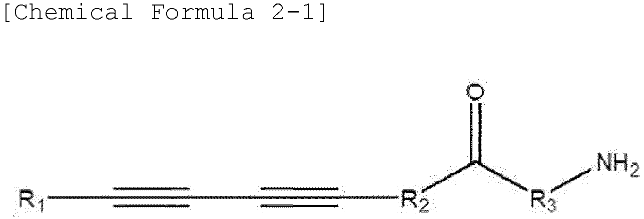

in Chemical Formula above, R₁ is a C₁-C₁₅ alkyl group, R₂ is a C₁-C₁₀ alkylene group, and R₃ is -NH-C₁-C₆ alkylene group or a C₁-C₆ alkylene group, preferably R₁ is a C₆-C₁₃ alkyl group, R₂ is a C₄-C₉ alkylene group, and R₃ is -NH-C₁-C₄ alkylene group, and more preferably R₁ is a C₁₀-C₁₂ alkyl group, R₂ is a C₆-C₈ alkylene group, and R₃ is -NH-C₁-C₃ alkylene group, 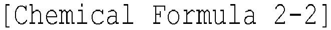 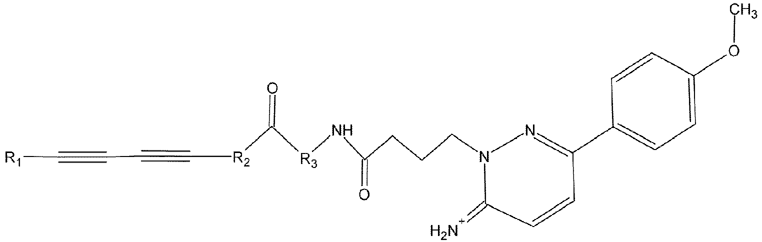 in Chemical Formula above, R₁ is a C₁-C₁₅ alkyl group, R₂ is a C₁-C₁₀ alkylene group, and R₃ is -NH-C₁-C₆ alkylene group or a C₁-C₆ alkylene group, preferably R₁ is a C₆-C₁₃ alkyl group, R₂ is a C₄-C₉ alkylene group, and R₃ is -NH-C₁-C₄ alkylene group, and more preferably R₁ is a C₁₀-C₁₂ alkyl group, R₂ is a C₆-C₈ alkylene group, and R₃ is -NH-C₁-C₃ alkylene group.

As used herein, the term "alkyl" refers to a linear or branched saturated aliphatic hydrocarbon group comprised of carbon and hydrogen atoms linked to the rest of the molecule through a single bond, and the term "C₁-C₁₅ alkyl" in the present disclosure means an alkyl group as defined above having at least one and up to 15 carbon atoms. Non-limiting examples of alkyl include methyl, ethyl, propyl, 2-propyl, n-butyl, isobutyl, t-butyl, n-pentyl, 2-methylbutyl, neopentyl, n-hexyl, 2-methylhexyl, -CH₂-cyclopropyl, and the like, but alkyl is not limited thereto. Preferably, the alkyl according to the present disclosure may be C₁₀ to C₁₅ straight chain or branched chain alkyl.

As used herein, the term "alkylene" refers to a linear or branched saturated aliphatic hydrocarbon radical comprised of carbon and hydrogen atoms linked to the rest of the molecule through a single bond, and the term "C₁-C₁₀ alkylene" in the present disclosure means an alkylene group as defined above having at least one and up to 10 carbon atoms. Non-limiting examples of alkylene include methylene, ethylene, propylene, 2-propylene, n-butylene, isobutylene, t-butylene, n-pentylene, 2-methylbutylene, neopentylene, n-hexylene, 2-methylhexylene, -CH₂-cyclopropylene, and the like, but alkylene is not limited thereto. Preferably, the alkylene according to the present disclosure may be C₁ to C₈ straight chain or branched chain alkylene.

As used herein, the term "illicit drug" may refer to a drug that is misused and abused beyond the scope of its medicinal use or a drug that is frequently illegal such as a narcotic. The illicit drug may include narcotics such as drug narcotics, psychotropic drugs, marijuana, and the like. The narcotics are classified into natural drugs (for example, poppy, opium, coca leaves), semi-synthetic drugs (for example, heroin), and synthetic drugs (for example, oxycodone, pethidine, methadone, fentanyl) depending on the type of raw plants and production method thereof. The psychotropic drugs act on the central nervous system of humans, and may include some stimulants, hallucinogens, depressants, anesthetics and tranquilizers.

Non-limiting examples of illicit drugs in the present disclosure include gamma-hydroxybutyrate (GHB), ketamine, rohypnol, philopon, ecstasy, zolpidem, cocaine, heroin, amphetamine, methamphetamine, LSD, cannabis, poppy, opiates, benzodiazepines, barbiturates, mescaline, psilocybin, oxycodone, pethidine, methadone, fentanyl, and the like, preferably gamma-hydroxybutyrate (GHB). GHB is a psychotropic drug and becoming a social problem since it is used as a date rape drug, an illicit drug that is abused for sexual crimes. It is known to be more dangerous because it is colorless and odorless, and has a salty taste. In addition, GHB has a short half-life due to drug characteristics, and thus when mixed with alcohol, GHB is excreted from the body through urine within 30 minutes to 1 hour 30 minutes. As described above, the present disclosure is significant in that there are provided a novel reagent composition and detection kit for easy and rapid detection of GHB used in violent crimes such as sexual crimes, and the like.

In the present disclosure, the reagent composition for detecting illicit drugs may comprise the diacetylene derivative compound represented by Chemical Formula 1; gabazine (SR-95531); a polyethylene glycol-based compound; and a thermoplastic fluorine polymer compound.

In the present disclosure, the reagent composition for detecting illicit drugs may comprise an organic solvent, preferably acetone and methylpyrrolidone (NMP).

In addition, the reagent composition for detecting illicit drugs may further comprise methanol.

In the present disclosure, gabazine (SR-95531) is a compound represented by the following Chemical Formula 5-1 or Chemical Formula 5-2, and may comprise a salt thereof or an isomer thereof. Gabazine is generally known as a drug acting as an antagonist at the γ-aminobutyric acid type A (GABAA) receptor, but in the present disclosure, the possibility of using gabazine as a detection compound for detecting a specific compound or illicit drug was confirmed.

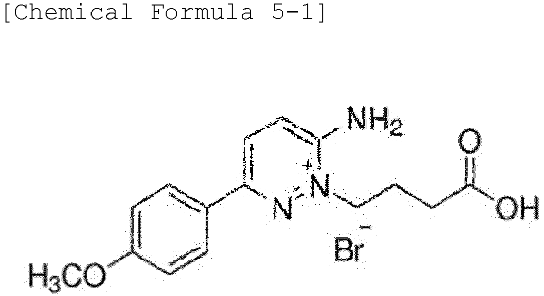

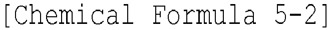

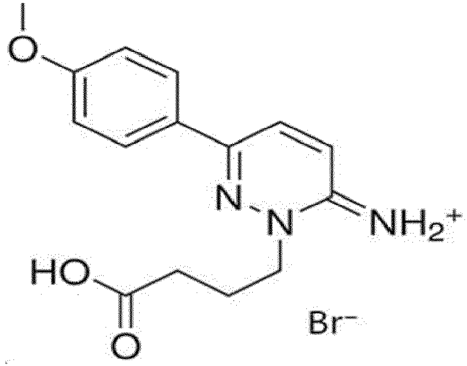

In the present disclosure, the diacetylene derivative compound represented by Chemical Formula 1 may be a compound represented by the following Chemical Formula 3:

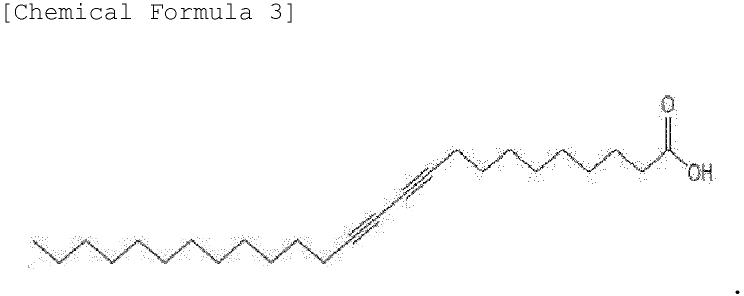

The compound represented by Chemical Formula 3 is named 10, 12-pentacosadiynoic acid, and is also referred to as "PCDA" in the present disclosure.

Further, in the present disclosure, the amine group-linked diacetylene derivative compound represented by Chemical Formula 2-1 may be a compound represented by the following Chemical Formula 4:

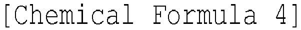

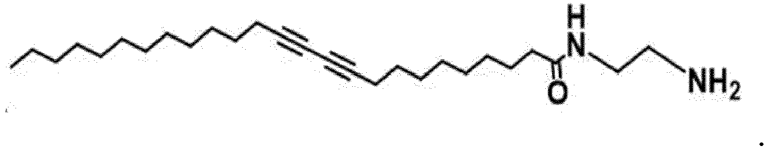

The compound represented by Chemical Formula 4 may be named N-(2-aminoethyl)pentacosa-10,12-diynamide, and for convenience, it is also referred to as "PCDA-NH₂" in the present disclosure. In the present disclosure, the amine group-linked diacetylene derivative compound (PCDA-NH₂) represented by Chemical Formula 2-1 is a compound in which PCDA and an amine compound containing at least two -NH₂ groups (for example, ethylenediamine) are linked by an amide bond, which is specifically obtained by reacting ethylenediamine with an intermediate compound (PCDA-NHS) obtained by esterification of -C(=O)OH of PCDA and -OH of N-hydroxysuccinimide (MHS). A specific synthesis method thereof was described in detail in Example 2 of the present specification.

Further, in the present disclosure, the gabazine-linked diacetylene derivative compound represented by Chemical Formula 2-2 may be a compound represented by the following Chemical Formula 6, and for convenience, it is also referred to as "PCDA-gabazine" in the present disclosure.

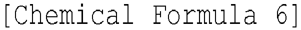

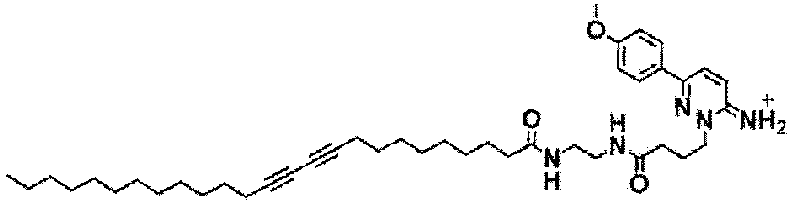

In the present disclosure, the polyethylene glycol-based compound and the thermoplastic fluorine polymer compound may further comprise a polyester-based compound or gelatin as necessary as an additive added to adjust physical properties of the composition or to have properties as a spinning solution for electrospinning. By adjusting the type and amount of additives, it is possible to adjust the physical properties of the reagent composition. In particular, the type and amount of additives may be selected to have optimal physical properties when preparing a spinning solution for electrospinning and to produce a sheet type by crosslinking.

In the present disclosure, the polyethylene glycol-based compound may include, but is not limited to, poly(ethylene oxide) (PEO), polyethylene glycol, and polyoxyethylene, and the like, and may preferably be poly(ethylene oxide) (PEO). The polyethylene oxide may have a weight average molecular weight (Mw) of 500 to 50,000, preferably 1,000 to 10,000, and more preferably 1,000 to 5,000.

In addition, the thermoplastic fluorine polymer compound may include, but is not limited to, polyvinylidene fluoride (PVDF), polytetrafluoroethylene (PTFE), polyvinylidene fluoride and polychlorotrifluoroethylene, and the like, and may preferably be polyvinylidene fluoride (PVDF). The polyvinylidene fluoride may have a weight average molecular weight (Mw) of at least 100,000, preferably at least 200,000, and more preferably at least 300,000 or at least 400,000.

Further, the polyester-based compound may include, but is not limited to, polycaprolactone (PCL), polyglycolide, poly(lactic acid), polyhydroxyalkanoates (PHAs), polyhydroxybutyrate, poly(lactic-co-glycolic acid), polybutylene succinate (PBS), and polyethylene terephthalate, and the like, and may preferably be polycaprolactone (PCL).

In the present disclosure, the reagent composition may contain, based on the total weight of the composition, 0.05 to 0.5 wt% of the diacetylene derivative compound represented by Chemical Formula 1; 0.05 to 0.5 wt% of gabazine; 0.5 to 5 wt% of the polyethylene glycol-based compound; and 5 to 20 wt% of the thermoplastic fluorine polymer compound.

Further, in the present disclosure, the reagent composition may contain, based on the total weight of the composition, 0.05 to 0.5 wt% of the diacetylene derivative compound represented by Chemical Formula 1; 0.01 to 0.2 wt% of the amine group-linked diacetylene derivative represented by Chemical Formula 2-1; 1 to 15 wt% of the polyethylene glycol-based compound; and 1 to 15 wt% of the thermoplastic fluorine polymer compound.

Further, in the present disclosure, the reagent composition may contain, based on the total weight of the composition, 0.05 to 0.5 wt% of the diacetylene derivative compound represented by Chemical Formula 1; 0.05 to 0.5 wt% of the gabazine-linked diacetylene derivative compound represented by Chemical Formula 2-2; 0.5 to 10 wt% of the polyethylene glycol-based compound; and 5 to 20 wt% of the thermoplastic fluorine polymer compound.

In addition, based on the total weight of the composition, the polyethylene glycol-based compound may preferably have an amount of 1 to 8 wt%, and more preferably 1 to 6 wt%.

In addition, the thermoplastic fluorine polymer compound may preferably have an amount of 5 to 15 wt%, and more preferably 5 to 13 wt%.

Further, the diacetylene derivative compound represented by Chemical Formula 1 may preferably have an amount of 0.05 to 0.4 wt%, and more preferably 0.1 to 0.3 wt%.

In addition, gabazine may preferably have an amount of 0.1 to 0.5 wt%, and more preferably 0.1 to 0.3 wt%.

Further, the amine group-linked diacetylene derivative compound represented by Chemical Formula 2-1 (PCDA-NH₂) may preferably have an amount of 0.01 to 0.1 wt%, and more preferably 0.03 to 0.1 wt%.

Further, the gabazine-linked diacetylene derivative compound represented by Chemical Formula 2-2 (PCDA-gabazine) may preferably have an amount of 0.01 to 0.1 wt%, and more preferably 0.02 to 0.07 wt%.

In another aspect of the present disclosure, there is provided a sheet kit for detecting illicit drugs comprising the reagent composition.

In the present disclosure, the sheet kit for detecting illicit drugs of the present disclosure is capable of quickly and easily detecting GHB (gamma-hydroxybutyrate) among the various illicit drugs described above.

In the present disclosure, the sheet kit may be produced in the form of a sheet of fibers obtained by electrospinning the reagent composition.

In the present disclosure, the sheet kit comprises the reagent composition defined above, wherein the diacetylene derivative compound represented by Chemical Formula 1 and/or the amine group-linked diacetylene derivative compound represented by Chemical Formula 2-1 (PCDA-NH₂) of the present disclosure contained in the reagent composition may have a diacetylene (-C≡C-C≡C-) structure, thereby being used for color analysis according to color change.

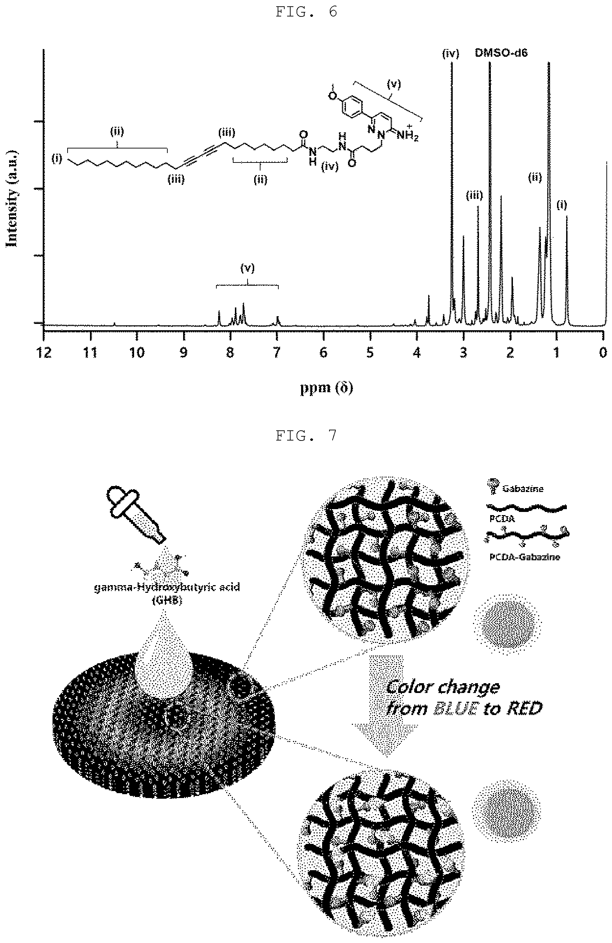

For example, when a drug to be analyzed (for example, illicit drug or narcotic drug) is incorporated into a sheet kit comprising the reagent composition of the present disclosure having a blue color, the side chain of the diacetylene derivative compound represented by Chemical Formula 1 and/or the amine group-linked diacetylene derivative compound represented by Chemical Formula 2-1 (PCDA-NH₂) of the present disclosure contained in the reagent composition may be disrupted. Since the incorporation changes the color to red by structural bonding with the diacetylene derivative compound and/or the amine group-linked diacetylene derivative compound (PCDA-NH₂) or the gabazine-linked diacetylene derivative compound (PCDA-gabazine), it is possible to detect the drug with the naked eye through color change (FIG. 7).

More specifically, when the reagent composition of the present disclosure is exposed to UV-light of 250 to 260 nm (specifically, 254 nm), polymerization between adjacent diacetylenes is generated and diacetylenes become blue. Accordingly, the diacetylene derivative compound or the amine group-linked diacetylene derivative compound (PCDA-NH₂) according to the present disclosure, which is a compound containing diacetylene, may change the color to red when stimulated by external stimuli such as temperature, pH, friction, surfactant, solvent, or binding of a target molecule (for example, narcotic drug), and the like. In other words, the color change (color transition) of the diacetylene derivative compound or the amine group-linked diacetylene derivative compound (PCDA-NH₂) may appear by structural bonding between the target molecule (for example, narcotics, drugs) and the conjugated backbone of the amine group-linked diacetylene derivative compound (PCDA-NH₂) induced by the external stimulus.

In still another aspect of the present disclosure, the present disclosure provides a method for producing a sheet kit for detecting illicit drugs, comprising: (S1) preparing a spinning solution containing the reagent composition according to the present disclosure; (S2) forming a sheet by electrospinning the spinning solution; and (S3) UV-treating the sheet.

The description of the reagent composition in step (S1) is the same as described above.

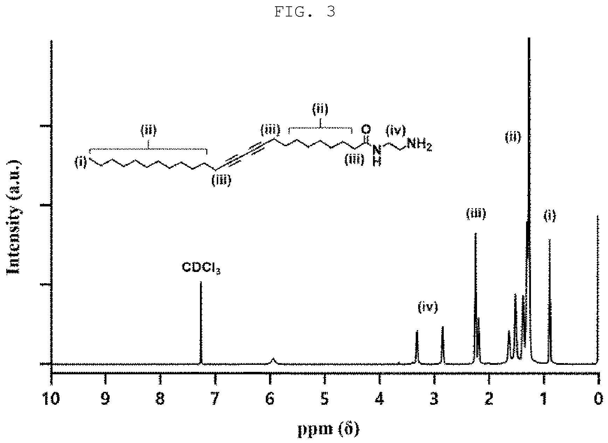

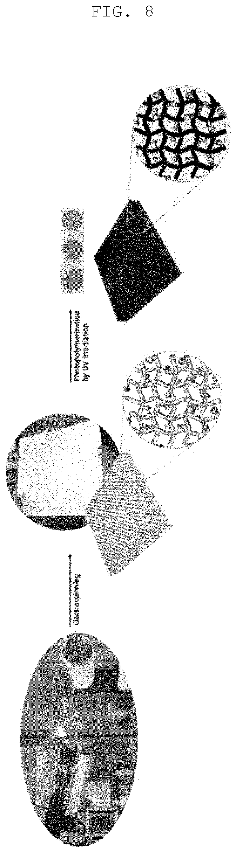

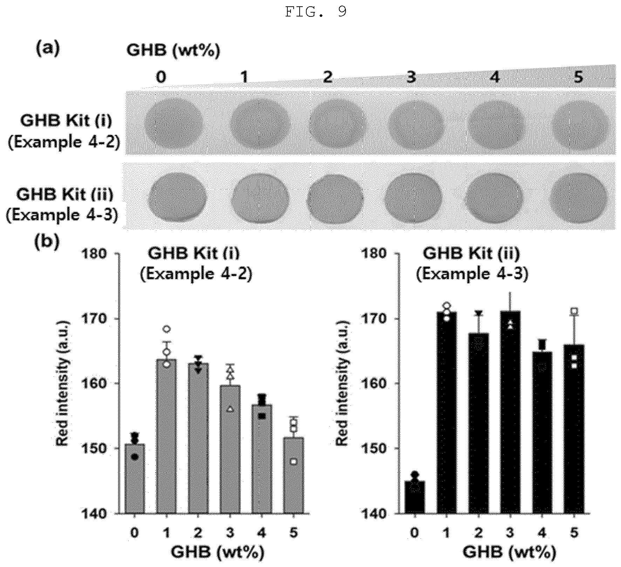

The production process of the sheet kit for detecting illicit drugs is as shown in FIGS. 3 and 9. Specifically, a reagent composition comprising polyethyleneoxide, polyvinylidene fluoride, 10,12-pentacosadiynoic acid (PCDA) and gabazine or a reagent composition comprising polyethyleneoxide, polyvinylidene fluoride, 10,12-pentacosadiynoic acid (PCDA) and PCDA-NH₂ is used as the spinning solution to form spun fibers (i.e., sheet) through electrospinning, and some or all of the prepared spun fibers are cross-linked by irradiating UV light, and thus a part irradiated with UV light appears blue. When a part showing blue color (that is, the part irradiated with UV light) is in contact with a drug, the color changes to red, allowing the drug to be detected (FIG. 8).

In the present disclosure, the spinning solution in step (S1) may further comprise a polyester-based compound or gelatin. The spinning solution is the same as described above as the reagent composition defined in the present disclosure.

In addition, in the present disclosure, the spinning solution may be prepared by mixing the reagent composition with a solvent for dissolving the reagent composition. In the present disclosure, water or an organic solvent may be used as the solvent for dissolving the reagent composition. Depending on the nature of the reagent composition to be dissolved, the solvent may be appropriately selected from water, a hydrophilic organic solvent, a hydrophobic organic solvent, and mixtures thereof, and may be acetone, methanol, ethanol, methylpyrrolidone (NMP), or the like. However, the kind of material usable as an organic solvent in the present disclosure is not particularly limited.

In the present disclosure, in step (S2), the electrospinning may be performed under the conditions that a distance between an electrode and a collector is 16 to 20 cm; an applied voltage is 15 to 25 kV; a temperature is 25 to 35°C; and a discharge rate of the spinning solution is 1 to 6 mL/hr. Preferably, a kit in the form of a sheet may be produced by electrospinning under the conditions of the distance between the electrode and the collector of 17 to 20 cm, the applied voltage of 15 to 22 kV, the temperature of 25 to 30°C, and the discharge rate of the spinning solution of 1 to 5 mL/hr.

With respect to the electrospinning in the present disclosure, when the voltage is less than 15 kV, sufficient electrostatic attraction/repulsion may not be generated due to the low voltage, and the spinning may fail, and when the voltage exceeds 25 kV, sparks may occur due to the high voltage. In addition, when the discharge rate is less than 1 mL/hr, there is a problem with productivity due to the excessively low speed of the spinning solution, and when the discharge rate exceeds 6 mL/hr, there is a possibility that fiberization may fail due to the excessively fast speed of the spinning solution. Further, it is possible to obtain fibers having various nano- or micro-sized diameters by adjusting the size of the inlet and outlet of the needle of the spinneret syringe (FIG. 12).

In the present disclosure, the sheet is capable of being produced by electrospinning using the reagent composition, thereby producing a flexible and sticker form that is able to be attached to the human body, wherein a size of the sheet is capable of being freely produced depending on the size of the object to be attached. Thus, the sheet of the present disclosure has a very high possibility of being used as a portable sheet for drug detection.

The present disclosure also provides a reagent composition for use in detecting illicit drugs, comprising: a diacetylene derivative compound represented by the following Chemical Formula 1; and at least one compound selected from the group consisting of gabazine (SR-95531), an amine group-linked diacetylene derivative represented by the following Chemical Formula 2-1, and a gabazine-linked diacetylene derivative represented by the following Chemical Formula 2-2:

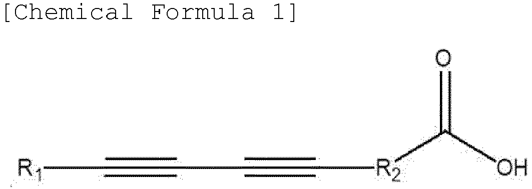

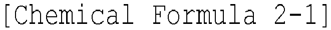

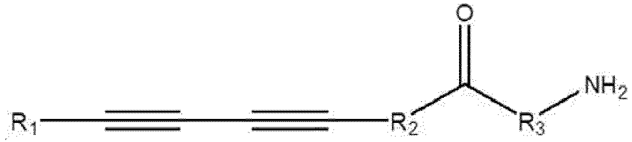

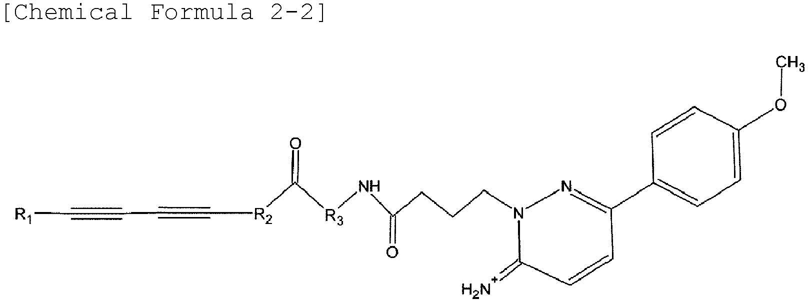

in Chemical Formula 1, Chemical Formula 2-1 or Chemical Formula 2-2 above,
R₁ is a C₁-C₁₅ alkyl group,
R₂ is a C₁-C₁₀ alkylene group, and
R₃ is -NH-C₁-C₆ alkylene group or a C₁-C₆ alkylene group.

The present disclosure also provides a reagent composition for use in detecting illicit drugs, comprising: a diacetylene derivative compound represented by the following Chemical Formula 1; at least one compound selected from the group consisting of gabazine (SR-95531), an amine group-linked diacetylene derivative represented by the following Chemical Formula 2-1, and a gabazine-linked diacetylene derivative represented by the following Chemical Formula 2-2; a polyethylene glycol-based compound; and a thermoplastic fluorine polymer compound. 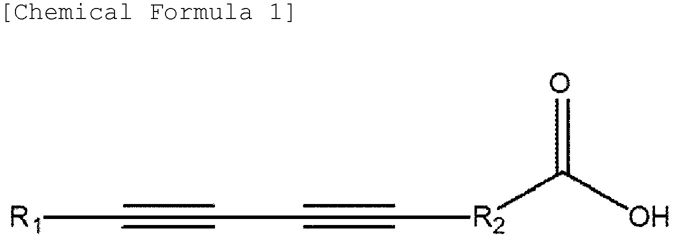 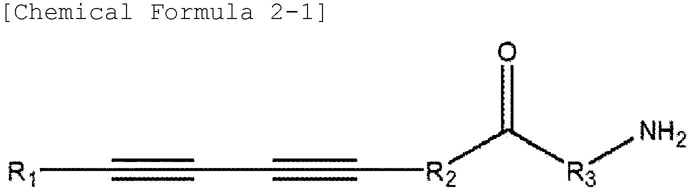 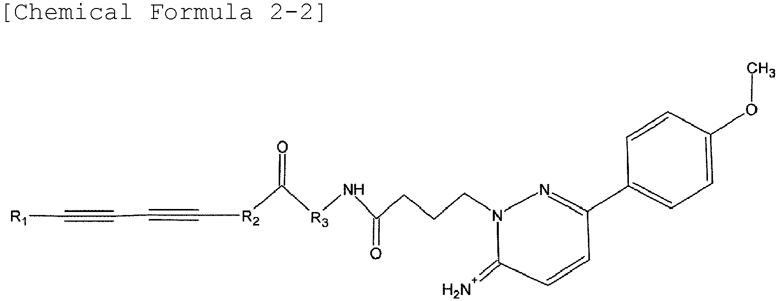
in Chemical Formula 1, Chemical Formula 2-1 or Chemical Formula 2-2 above,
R₁ is a C₁-C₁₅ alkyl group,
R₂ is a C₁-C₁₀ alkylene group, and
R₃ is -NH-C₁-C₆ alkylene group or a C₁-C₆ alkylene group.

In the preparation of a reagent for detecting illicit drugs, the present disclosure also provides use of a reagent composition comprising: the diacetylene derivative compound represented by Chemical Formula 1; and at least one compound selected from the group consisting of gabazine (SR-95531), the amine group-linked diacetylene derivative represented by Chemical Formula 2-1, and the gabazine-linked diacetylene derivative represented by Chemical Formula 2-2.

In the preparation of a reagent for detecting illicit drugs, the present disclosure also provides use of a reagent composition comprising: the diacetylene derivative compound represented by Chemical Formula 1; at least one compound selected from the group consisting of gabazine (SR-95531), the amine group-linked diacetylene derivative represented by Chemical Formula 2-1, and the gabazine-linked diacetylene derivative represented by Chemical Formula 2-2; the polyethylene glycol-based compound; and the thermoplastic fluorine polymer compound.

In addition, the present disclosure provides a method for detecting illicit drugs, comprising: contacting a detection sample with the sheet kit for detecting illicit drugs containing the reagent composition comprising: the diacetylene derivative compound represented by Chemical Formula 1; and at least one compound selected from the group consisting of gabazine (SR-95531), the amine group-linked diacetylene derivative represented by Chemical Formula 2-1, and the gabazine-linked diacetylene derivative represented by Chemical Formula 2-2.

Further, the present disclosure provides a method for detecting illicit drugs, comprising: contacting a detection sample with the sheet kit for detecting illicit drugs containing the reagent composition comprising: the diacetylene derivative compound represented by Chemical Formula 1; at least one compound selected from the group consisting of gabazine (SR-95531), the amine group-linked diacetylene derivative represented by Chemical Formula 2-1, and the gabazine-linked diacetylene derivative represented by Chemical Formula 2-2; the polyethylene glycol-based compound; and the thermoplastic fluorine polymer compound.

According to the present disclosure, it may be confirmed that when a detection sample containing no illicit drugs is in contact with the sheet containing the reagent composition, no color change occurs, but a detection sample containing an illicit drug is in contact with the sheet containing the reagent composition, color change occurs (color change is observed). Therefore, the sheet for detecting illicit drugs according to the present disclosure may easily check the presence or absence of illicit drugs through color change.

In the present disclosure, definitions of Chemical Formula 1, Chemical Formula 2-1 or Chemical Formula 2-2, gabazine, the polyethylene glycol-based compound, and the thermoplastic fluoropolymer compound are the same as described above.

### [Advantageous Effects]

The reagent composition for detecting illicit drugs of the present disclosure is capable of detecting drugs through color change in response to even a small amount of drug, and may be used to detect illicit drugs abused for sexual crimes.

In addition, it is possible to produce the composition in the form of a sheet by electrospinning, and thus the sheet is produced by a relatively simple process and is easy to carry, thereby making it possible to quickly and easily detect illicit drugs, especially GHB.

### [Description of Drawings]

FIG. 1 shows an NMR analysis result of gabazine prepared according to Example 1 of the present disclosure.
FIG. 2 shows a FT-IR analysis result of PCDA-NH₂ prepared according to Example 2-1 of the present disclosure.
FIG. 3 shows an NMR analysis result of PCDA-NH₂ prepared according to Example 2-1 of the present disclosure.

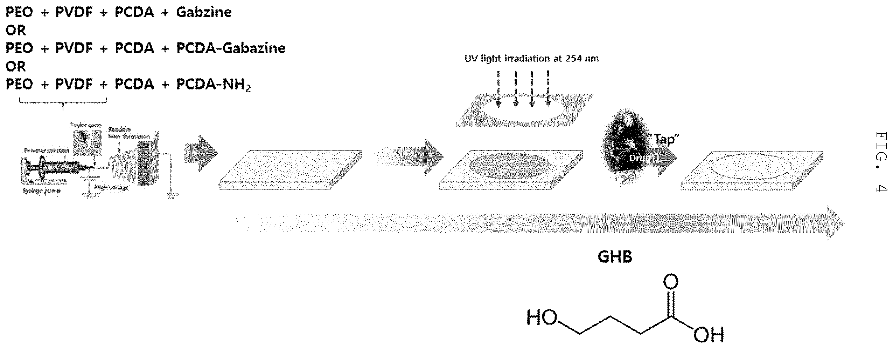

FIG. 4 is a schematic diagram showing a production process of a sheet kit containing a reagent composition according to the present disclosure.
FIG. 5 shows a detection result of GHB in the sheet kit containing the reagent composition of Example 4-1 according to the present disclosure.
FIG. 6 shows an NMR analysis result of PCDA-gabazine prepared according to Example 2-2 of the present disclosure.
FIG. 7 is a schematic diagram showing a sheet kit for sensing GHB containing the reagent composition of the present disclosure.
FIG. 8 shows a process for producing a sheet kit containing the reagent composition of the present disclosure through electrospinning.
FIG. 9 shows GHB detection results of the sheet for drug detection of Examples 4-2 and 4-3 according to the present disclosure.
FIG. 10 shows the detection results of GHB dissolved in various beverages of the sheet for drug detection of Example 4-3 according to the present disclosure.
FIG. 11 shows a SEM image of the sheet for drug detection produced according to the present disclosure.

### [Best Mode]

Hereinafter, the present disclosure will be described in more detail through Examples. However, these Examples are provided to illustrate the present disclosure by way of example, and the scope of the present disclosure is not limited to these Examples.

### Example 1: Synthesis of gabazine

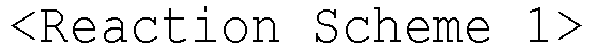

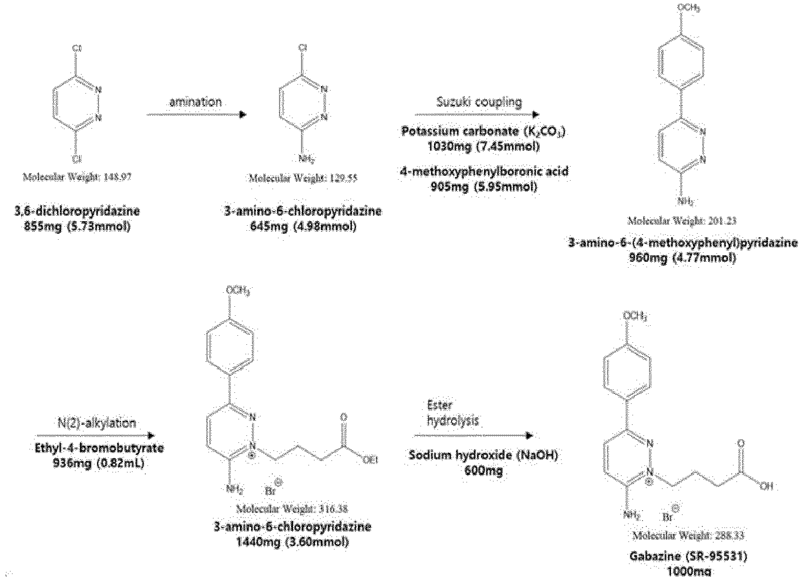

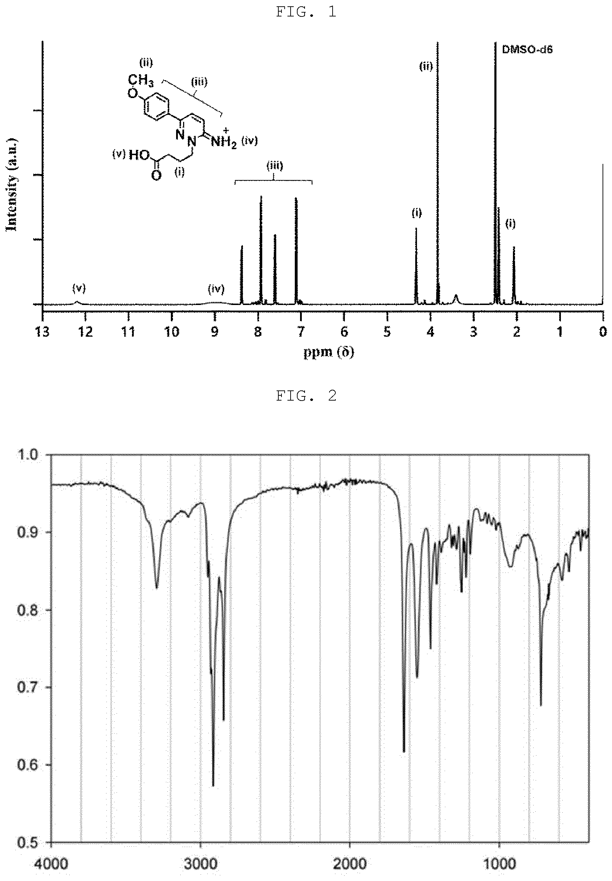

Gabazine was synthesized in the same order as shown in Reaction Scheme 1 above by a method described in the document "Org. Biomol. Chem., 2010, 8, 4131-4136". The structure of the prepared gabazine was confirmed through NMR analysis, and results thereof are shown in FIG. 1.

### Example 2: Preparation of diacetylene derivative compound

### Example 2-1: Synthesis of amine group-linked diacetylene derivative compound (PCDA-NH₂)

### (Step 1) Synthesis of PCDA-NHS

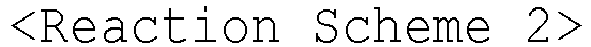

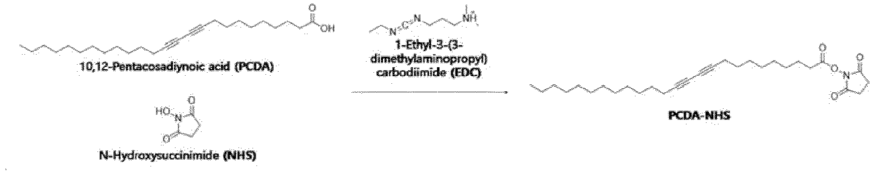

A solution was prepared by dissolving 10,12-pentacosadiynoic acid (PCDA) (2.67 mmol, 1 g) and 1-ethyl-3-[3-dimethyl-aminopropyl]carbodiimide hydrochloride (EDC) (4 mmol, 620 mg) in 50 mL of dichloromethane (DCM). To the prepared solution, N-hydroxysuccinimide (NHS) (4 mmol, 460 mg) was added, and the mixture was stirred at room temperature for 8 hours. After the reaction, the organic solvent was removed by evaporation under a vacuum atmosphere, and then the crude product was poured into distilled water and extracted three times with ethyl acetate for purification. Then, the organic solvent was dried over anhydrous magnesium sulfate and concentrated in vacuum to obtain a white powder.

It was confirmed through thin layer chromatography (TLC, hexane/ethyl acetate, 3:1) and 1H-NMR (600 MHz, DMSO-d6) that the white powder obtained after concentration was PCDA-NHS. ¹H-NMR peak values of the prepared PCDA-NHS are as follows.

¹H-NMR(600MHz) δ(ppm) : 0.86 (t, 3H), 1.24-1.62 (m, 32H), 2.27 (t, 4H), 2.65 (t, 2H), 2.81 (s, 4H).

### (Step 2) Synthesis of PCDA-NH₂

A solution containing 50 mL of dichloromethane and PCDA-NHS (1 mmol, 472 mg) prepared in step 1 was slowly added dropwise to 50 mL of dichloromethane solution containing ethylenediamine (EDA) (50 mmol, 3 g), and the mixture was stirred at room temperature overnight. After the reaction, the organic solvent was removed through filtration, and the resulting product was purified by extraction three times using water and dichloromethane. Subsequently, the organic solvent was removed using anhydrous magnesium sulfate and the resulting product was concentrated in vacuum to obtain PCDA-NH₂ in the form of a light-blue powder.

It was confirmed through thin layer chromatography (TLC, methanol/ethyl acetate, 3:7), FT-IR, and 1H-NMR (600 MHz, DMSO-d6) that the obtained light-blue powder was PCDA-NH₂. 1H-NMR analysis results of the prepared PCDA-NH₂ are shown in FIG. 3, and the peak values are as follows. In addition, the FT-IR analysis results of PCDA-NH₂ are shown in FIG. 2.

¹H-NMR(600 MHz) δ(ppm) : 0.88 (t, 3H), 1.26-1.63 (m, 32H), 2.18 (t, 2H), 2.24 (t, 4H), 2.83 (m, 2H), 3.30 (q, 2H), 5.86 (brs, 1H)

### Example 2-2: Synthesis of gabazine-linked diacetylene derivative compound (PCDA-gabazine)

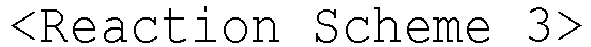

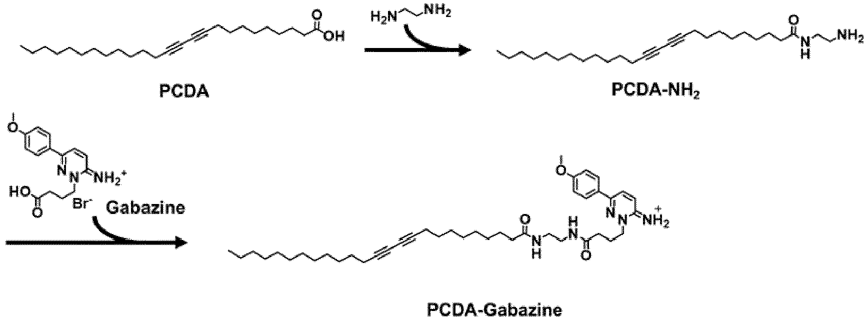

442 mg (1.2 mmol) of Gabazine prepared according to Example 1 was dissolved in 20 mL of dimethylformamide (DMF). Then, 372 mg (2.4 mmol) of 1-ethyl-3-[3-dimethylaminopropyl]carbodiimide hydrochloride (EDC), and 276 mg (2.4 mmol) of N-hydroxysuccinimide (NHS) were added, followed by stirring at room temperature for 8 hours to proceed with the reaction. Then, 419 mg (1 mmol) of PCDA-NH₂ prepared according to Example 2-1 was added to the reaction mixture and stirred overnight at room temperature to prepare a mixture. Then, for purification, the mixture was extracted with ethyl acetate and DMF was removed with distilled water (3 times). Then, the organic solvent was dried over anhydrous magnesium sulfate and concentrated in vacuum to obtain a light-blue powder.

It was confirmed through thin layer chromatography (TLC, methanol/ethyl acetate, 3:7), and 1H-NMR spectroscopy (600 MHz, CDCl₃) that the obtained light-blue powder was PCDA-gabazine.

¹H-NMR analysis results of the prepared PCDA-gabazine are shown in FIG. 7, and specific peak values are as follows.

¹H-NMR(600 MHz) δ(ppm) : 0.85 (t, 3H), 1.24-1.44 (m, 32H), 2.03 (t, 2H), 2.27 (t, 4H), 2.38 (t, 2H), 2.76 (m, 2H), 3.07 (m, 2H), 3.31 (d, 2H), 3.81 (s, 3H), 7.03-7.15 (m. 2H), 7.77-7.85 (m. 2H), 7.94-8.01 (m, 4H), 8.30 (m, 1H).

### Example 3: Preparation of reagent composition for detecting illicit drugs

### Example 3-1: Preparation of Reagent Composition 1 (PCDA+PEO+PVDF+PCDA-NH₂)

450 mg of Polyvinylidene fluoride (PVDF) having a weight average molecular weight of 400,000 was dissolved in a mixed solvent containing 1.28 g of N-methyl-2-pyrrolidone (NMP) and 5.98 g of acetone, and 450 mg of poly(ethylene oxide) (PEO) having a weight average molecular weight of 2000 to 3000 was added thereto. After mixing thoroughly, 18 mg of 10,12-pentacosadiynoic acid (PCDA) and 4.5 mg of PCDA-NH₂ prepared according to Example 2-1 were added and mixed to be dispersed well, thereby preparing Reagent Composition 1.

### Example 3-2: Preparation of Reagent Composition 2 (PCDA+PEO+PVDF+gabazine)

810 mg of Polyvinylidene fluoride (PVDF) having a weight average molecular weight of 400,000 and 90 mg of poly(ethylene oxide) (PEO) having a weight average molecular weight of 2000 to 3000 were dissolved in 1.28 g of N-methyl-2-pyrrolidone (NMP) to prepare a matrix solution to be used as a matrix layer.

In addition, 11.25 mg of 10,12-pentacosadiynoic acid (PCDA) and 11.25 mg of gabazine prepared according to Example 1 were dissolved in a mixed solvent containing 4.5 g of acetone and 0.16 ml of methanol to prepare a sensing solution.

The prepared matrix solution and sensing solution were uniformly mixed at a temperature of 40°C, thereby preparing Reagent Composition 2.

### Example 3-3: Preparation of Reagent Composition 3 (PCDA+PEO+PVDF+PCDA-gabazine)

600 mg of Polyvinylidene fluoride (PVDF) having a weight average molecular weight of 400,000 and 300 mg of poly(ethylene oxide) (PEO) having a weight average molecular weight of 2000 to 3000 were dissolved in 1.7 g of N-methyl-2-pyrrolidone (NMP) to prepare a matrix solution to be used as a matrix layer.

In addition, 18 mg of 10,12-pentacosadiynoic acid (PCDA) and 4.50 mg of PCDA-gabazine prepared according to Example 2-2 were dissolved in 4.9 g of acetone to prepare a sensing solution.

The prepared matrix solution and sensing solution were uniformly mixed at a temperature of 40°C, thereby preparing Reagent Composition 3.

### Example 4: Production of sheet for detecting illicit drugs using electrospinning

### Example 4-1: Production of sheet for drug detection comprising reagent composition 1

The reagent composition prepared according to Example 3-1 was subjected to electrospinning. When electrospinning the prepared solution, a sheet paper was produced by electrospinning for 2 hours under conditions that a distance between an electrode and a collector is 18.5 cm, an applied voltage is 20 kV, a temperature is 28°C, and a discharge rate of a spinning solution is 3 mL/hr. The produced sheet was processed into various sizes and irradiated with 254 nm UV for 10 seconds to produce a sheet for detecting illicit drugs.

### Example 4-2: Production of sheet for drug detection comprising Reagent Composition 2

The reagent composition 2 prepared according to Example 3-2 was subjected to electrospinning. When electrospinning the prepared solution, a sheet paper was produced by electrospinning for 2 hours under conditions that a distance between an electrode and a collector is 18.5 cm, an applied voltage is 20 kV, a temperature is 28°C, and a discharge rate of a spinning solution is 3 mL/hr. The produced sheet was processed into various sizes and irradiated with 254 nm UV for 10 seconds to produce a sheet for detecting illicit drugs.

### Example 4-3: Production of sheet for drug detection comprising Reagent Composition 3

The reagent composition 3 prepared according to Example 3-3 was subjected to electrospinning. When electrospinning the prepared solution, a sheet paper was produced by electrospinning for 2 hours under conditions that a distance between an electrode and a collector is 18.5 cm, an applied voltage is 20 kV, a temperature is 28°C, and a discharge rate of a spinning solution is 3 mL/hr. The produced sheet was processed into various sizes and irradiated with 254 nm UV for 10 seconds to produce a sheet for detecting illicit drugs.

### Example 5: Drug detection using sheet for drug detection of the present disclosure

### Example 5-1: GHB detection characteristics according to GHB content

The illicit drug GHB (legally obtained with permission from the Ministry of Food and Drug Safety (MFDS); Permission No. 443) was dissolved in water to prepare samples of 1 to 5% concentration, respectively, and water in which no illicit drugs were dissolved was used as a control group (0%).

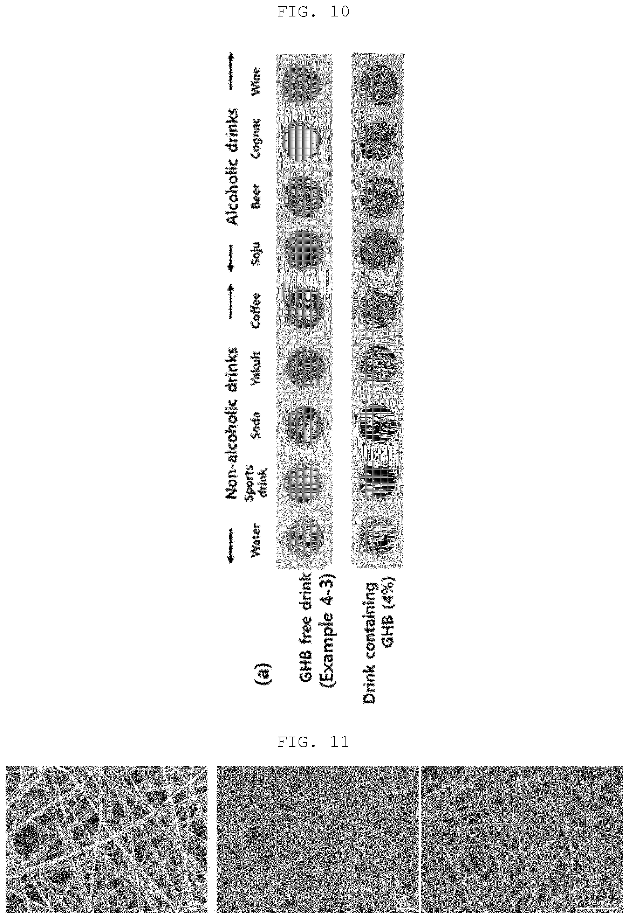

Each of the prepared 1% to 5% sample and the control group (0%) was dropped on the sheets produced according to Example 4-2 and Example 4-3, and the color change depending on the drug concentration was observed. Results thereof are shown in FIG. 10.

Referring to FIG. 9, it could be confirmed that when the control group (0%) was in contact with the sheet containing the reagent composition according to the present disclosure, no color change occurred, whereas when the sample containing the GHB drug was in contact with the sheet, the color change from blue to red occurred even at the GHB concentration of 1%. In particular, as compared to the control group (water; 0%), the sheet containing the reagent composition 3 of the present disclosure (Example 4-3) showed a significant increase in red intensity when contacted with GHB regardless of the concentration of the GHB drug.

Therefore, the sheet for detecting illicit drugs according to the present disclosure may easily check the presence or absence of illicit drugs through color change.

### Example 5-2: GHB detection characteristics according to the type of solvent in which GHB is dissolved

Since GHB has colorless, odorless, and tasteless characteristics, it is not easy to ascertain its existence, and especially when mixed with various non-alcoholic or alcoholic beverages, it is more difficult to confirm its existence. Therefore, the ability of the sheet of the present disclosure to detect GHB was evaluated using various beverages containing GHB as solvents.

Water, sports drinks, carbonated drinks, yogurt (Yakult) and coffee were used as non-alcoholic beverages, soju, beer, cognac, and wine were used as alcoholic beverages. Then, GHB, an illicit drug, was dissolved in the above-described beverages as solvents to prepare samples at a concentration of 4 to 5%, respectively, and a sample in which the illicit drug was not dissolved was used as a control group (without GHB or GHB free drink).

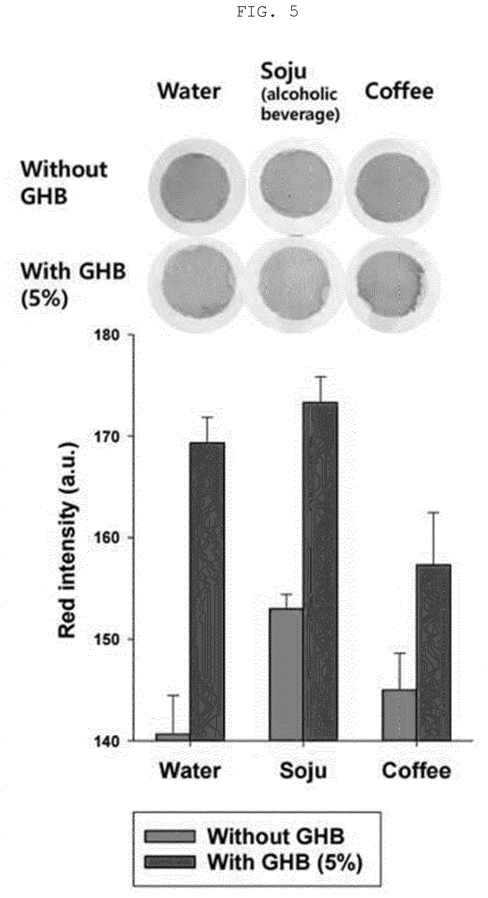

Color change according to the type of solvent in which the drug was dissolved was observed by dropping each of the prepared samples and control group on sheets produced according to Example 4-1 and Example 4-3. Results thereof are shown in FIGS. 5 and 10.

Referring to FIGS. 5 and 10, it could be confirmed that the color of the sheet changed from blue to red regardless of the type of solvent in which GHB was dissolved. In general, GHB is known to occur naturally in red wine by fermentation of red grapes (4.1 to 21.4 mg/L), and even in the absence of GHB, various additives in beverages may cause unintended color changes. However, the sheet containing the reagent composition of the present disclosure showed a clear color change when GHB was present even in a small amount, and thus the presence or absence of GHB could be easily confirmed with the naked eye without the use of sophisticated analysis equipment. Also, as time passed, the red color became more vivid and the intensity increased.

These results suggest that if the illicit drug GHB is present, the reagent composition of the present disclosure is able to easily detect the drug through color change regardless of the small amount of the drug or the type of solvent in which the illicit drug is dissolved.

From the above description, those skilled in the art to which the present disclosure pertains will understand that the present disclosure may be embodied in other specific forms without changing the technical spirit or essential characteristics thereof. In this regard, it should be understood that the embodiments described above are illustrative in all respects and not restrictive. The scope of the present disclosure should be construed as including all changes or modifications derived from the meaning and scope of the claims to be described below and equivalent concepts rather than the detailed description above are included in the scope of the present disclosure.

## Claims

1. A reagent composition for detecting illicit drugs, comprising:
a diacetylene derivative compound represented by the following Chemical Formula 1; and
at least one compound selected from the group consisting of gabazine (SR-95531), an amine group-linked diacetylene derivative represented by the following Chemical Formula 2-1, and a gabazine-linked diacetylene derivative represented by the following Chemical Formula 2-2, and
optionally, further comprising or not comprising a polyethylene glycol-based compound; and a thermoplastic fluorine polymer compound: 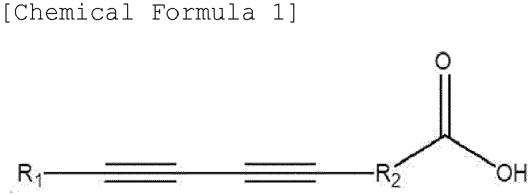 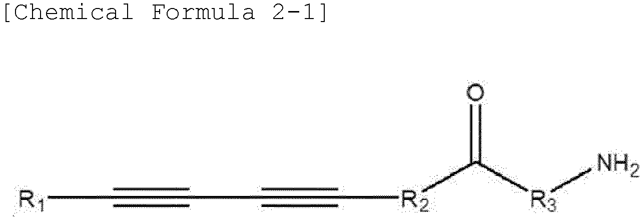 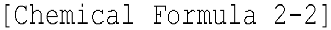 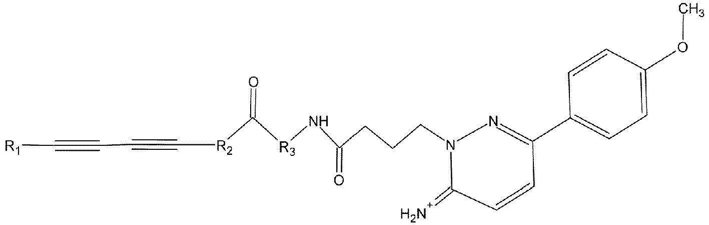
in Chemical Formula 1, Chemical Formula 2-1 or Chemical Formula 2-2 above,
R₁ is a C₁-C₁₅ alkyl group,
R₂ is a C₁-C₁₀ alkylene group, and
R₃ is -NH-C₁-C₆ alkylene group or a C₁-C₆ alkylene group.

2. The reagent composition of claim 1, wherein the reagent composition comprises:
the diacetylene derivative compound represented by Chemical Formula 1;
gabazine (SR-95531);
the polyethylene glycol-based compound; and
the thermoplastic fluorine polymer compound.

3. The reagent composition of claim 1, wherein the reagent composition comprises:
the diacetylene derivative compound represented by Chemical Formula 1;
the amine group-linked diacetylene derivative represented by Chemical Formula 2-1;
the polyethylene glycol-based compound; and
the thermoplastic fluorine polymer compound.

4. The reagent composition of claim 1, wherein the reagent composition comprises:
the diacetylene derivative compound represented by Chemical Formula 1;
the gabazine-linked diacetylene derivative represented by the following Chemical Formula 2-2;
the polyethylene glycol-based compound; and
the thermoplastic fluorine polymer compound.

5. The reagent composition of claim 1, wherein the reagent composition further comprises a polyester-based compound or gelatin, and
the polyester-based compound is at least one selected from the group consisting of polycaprolactone (PCL), polyglycolide, poly(lactic acid), polyhydroxyalkanoate (PHA), polyhydroxybutyrate, poly(lactic-co-glycolic acid), polybutylene succinate (PBS), and polyethylene terephthalate.

6. The reagent composition of claim 1, wherein the diacetylene derivative compound represented by Chemical Formula 1 is a compound represented by the following Chemical Formula 3,
the amine group-linked diacetylene derivative represented by Chemical Formula 2-1 is a compound represented by the following Chemical Formula 4, and
the gabazine-linked diacetylene derivative represented by the following Chemical Formula 2-2 is a compound represented by the following Chemical Formula 6:

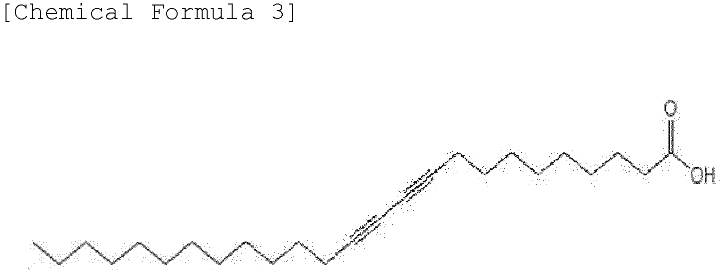

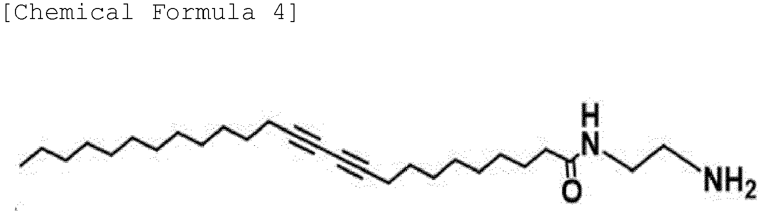

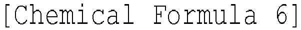

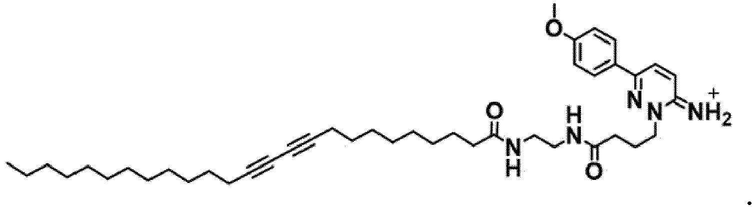

7. The reagent composition of claim 1, wherein the reagent composition comprises, based on the total weight of the composition, 0.05 to 0.5 wt% of the diacetylene derivative compound represented by Chemical Formula 1;
0.05 to 0.5 wt% of gabazine;
0.5 to 5 wt% of the polyethylene glycol-based compound; and
5 to 20 wt% of the thermoplastic fluorine polymer compound.

8. The reagent composition of claim 1, wherein the reagent composition comprises, based on the total weight of the composition, 0.05 to 0.5 wt% of the diacetylene derivative compound represented by Chemical Formula 1;
0.01 to 0.2 wt% of the amine group-linked diacetylene derivative represented by Chemical Formula 2-1;
1 to 15 wt% of the polyethylene glycol-based compound; and
1 to 15 wt% of the thermoplastic fluorine polymer compound.

9. The reagent composition of claim 1, wherein the reagent composition comprises, based on the total weight of the composition, 0.05 to 0.5 wt% of the diacetylene derivative compound represented by Chemical Formula 1;
0.01 to 0.1 wt% of the gabazine-linked diacetylene derivative compound represented by Chemical Formula 2-2;
0.5 to 10 wt% of the polyethylene glycol-based compound; and
5 to 20 wt% of the thermoplastic fluorine polymer compound.

10. The reagent composition of claim 1, wherein the polyethylene glycol-based compound is at least one selected from the group consisting of poly(ethylene oxide) (PEO), polyethylene glycol, and polyoxyethylene, and
the thermoplastic fluorine polymer compound is at least one selected from the group consisting of polyvinylidene fluoride (PVDF), polytetrafluoroethylene (PTFE), polyvinylidene fluoride, and polychlorotrifluoroethylene.

11. The reagent composition of claim 1, wherein the illicit drug is selected from the group consisting of gamma-hydroxybutyric acid (GHB), ketamine, rohypnol, philopon, ecstasy, zolpidem, cocaine, and heroin.

12. A method for producing a sheet kit for detecting illicit drugs, comprising:
(51) preparing a spinning solution containing the reagent composition of claim 1;
(S2) forming a sheet by electrospinning the spinning solution; and
(S3) UV-treating the sheet.

13. The method of claim 12, wherein in step (S2), the electrospinning is performed under the conditions that a distance between an electrode and a collector is 16 to 20 cm; an applied voltage is 15 to 25 kV; a temperature is 25 to 35°C; and a discharge rate of the spinning solution is 1 to 6 mL/hr.

14. The method of claim 12, wherein the illicit drug is selected from the group consisting of gamma-hydroxybutyric acid (GHB), ketamine, rohypnol, philopon, ecstasy, zolpidem, cocaine, and heroin.

15. A method for detecting illicit drugs, comprising:
contacting a detection sample with the reagent composition for detecting illicit drugs according to claim 1; or the sheet kit for detecting illicit drugs produced according to claim 12.

16. The method of claim 15, wherein a color change is observed in the reagent composition for detecting illicit drugs or the sheet kit for detecting illicit drugs when the detection sample contains illicit drugs.

17. The method of claim 15, wherein the illicit drug is selected from the group consisting of gamma-hydroxybutyric acid (GHB), ketamine, rohypnol, philopon, ecstasy, zolpidem, cocaine, and heroin.
